Europäisches Patentamt

European Patent Office

Office européen des brevets

(11) Publication number: **0 201 075**
**A1**

## EUROPEAN PATENT APPLICATION

(21) Application number: **86106163.8**

(22) Date of filing: **06.05.86**

(51) Int. Cl.⁴: **A61F 11/00 , A61F 2/18**

(30) Priority: **08.05.85 ES 286591 U**
**23.04.86 ES 293768 U**

(43) Date of publication of application:
**17.12.86 Bulletin 86/46**

(84) Designated Contracting States:
**AT BE CH DE FR GB IT LI LU NL SE**

(71) Applicant: **MURUETA-GOYENA MENDIZABAL,**
**Federico**
**C/Negubide 7**
**Las Arenas Vizcaya(ES)**
Applicant: **Scola Basaguren, Esteban**
**C/Maria de Molina, 12**
**Madrid(ES)**

(72) Inventor: **MURUETA-GOYENA MENDIZABAL,**
**Federico**
**C/Negubide 7**
**Las Arenas Vizcaya(ES)**
Inventor: **Scola Basaguren, Esteban**
**C/Maria de Molina, 12**
**Madrid(ES)**

(74) Representative: **Prato, Roberto et al**
**c/o Ingg. Carlo e Mario Torta Via Viotti 9**
**I-10121 Torino(IT)**

(54) A tympanic isopressor for the treatment of the hypacusia.

㊼ A tympanic isopressor is described, comprising an air compressor (M) having a low capacity, an air reservoir (A), an applicator nozzle (15), a pressure regulator (V), an air filter (F) and a microcomputer. By means of the device according to the present invention, the administration of a treatment is obtained at a constant pressure measurable at any time in a simple and safe way.

FIG. 1

## "A TYMPANIC ISOPRESSOR FOR THE TREATMENT OF THE HYPACUSIA"

The present invention relates to a tympanic isopressor intended for the treatment of the hypacusia (deafness) of the acoustical transmission (middle ear).

Till now, this treatment was performed by means of an enema or rubber-syringe of Politzer, which was invented in the past century by the Viennese otorhinolaryngologist of the same name. With this device it was tried to attain the proper pressure, substantially through the Eustachian tube, into the eardrum.

Said device is based on the use of a rubber-syringe through the compression of which a supply of air at a fixed pressure, depending on the force by which the rubber-syringe is distorted, is obtained. As a consequence the above-mentioned device has the drawback that it supplies a discontinuous pressure, low and uncontrollable and not measurable, being applied manually. Therefore it is practically impossible the introduction into the Eustachian tube of a continuous appropriate treatment.

The object of the present invention is to provide a device having a simple construction, operation and handling, so that it can be used by the otorhinolaryngologist or by the patient himself. Further, the device according to the invention has a reduced cost and its operation is fast and effective.

By means of the isopressor according to the invention the administration is obtained at a constant pressure, measurable at any time, and which allows the administrtion or the removal at any time of the dressing.

According to the present invention, the isopressor comprises an air compressor having a low capacity, driven by an electric motor; a tubular tank for the compressed air, coupled with the compressor, and an outlet opening connected with the tank.

The compressor, besides being of low capacity, should not work with oil in order to avoid iatrogenic problems (avoid the introduction of oil droplets into the ear).

Another object of the present invention is to realize an isopressor having subsidiary elements, comprising a microcomputer, that enable the increase of the performance, support the applied treatment and simplify the handling.

The isopressor according to the invention comprises, in the air inlet to the compressor, a filter for the particles, and the outlet of said compressor comprises another filter of the dry type, for the removal of solid and liquid particles.

The compressor is fed by electric energy from the domestic network and is of the membrane type, in order to avoid the possible existence of oil, that can be dangerous for the patient to be treated. On the other hand, the membrane-type compressors usually have a low pressure (3.5 kg/cm² max.), which results in a lower risk for the patients and the user, in case of a failure of the pressure control.

The filter at the outlet from the compressor includes a blow-off valve to eliminate the water collected into the filter during the filtrtion, which is accessible from outside the equipment so to eliminate the liquid.

Through the dry filter, air is introduced in a reservoir placed for this, whose inner pressure increases as the compressor operates.

In the air reservoir three air intakes are provided:

a) The first is connected to a pressure sensor or pressure transductor, which converts the pressure measurement into an electric measurement.

b) The second is connected to a variable pressure regulator, which subject to the position in which it is placed keeps the pressure into the reservoir, allowing the scavenging of the excess air in order to maintain a constant pressure, by means of an exhaust valve. Its adjustment is done from the exterior of the equipment.

(c) The third intake forms the air outlet for its use in the medical treatment and includes a solenoid valve, that is a key with electric actuation. This valve allows the exit of the air from the reservoir. The outflow will be continuous if the valve is kept permanently open, while it will be instantaneous if the valve is opened for a short time interval. The two operational ways of the isopressor are determined by the actuation of this valve.

The air outlet from the reservoir is connected from the control panel of the device or front of the same to the applicator which is formed by a small deposit or accumulator which accomplishes the double function of applicator and pressure regulator. At the opposite end of the connection of the air pipe, there is a restriction upon which the detachable nozzle for the direct application to the patient is placed. Above the casing of the applicator is placed a push button which when actuated causes the opening of the discharge valve in the form an istantaneous discharge operation.

For a precise control of the operation of the device a microcomputer is used as the most modern and dependable electronic means, which carries out the control functions aided by the complementary circuitry as described in the following:

1. It interprets a signal processed from the pressure transductor and converts it into a pressure measurement in the reservoir, presenting the value of the same with at least two figures on the front panel by means of LED numerical indicators, liquid crystals or the like. The resolution in this case is 0.1 kg/cm$^2$ though the precision is much higher. Analog-to-digital converters are used which support the high precision obtained.

2. To the microcomputer arrives one of the two operation instructions in one of the two ways of discharge: continuous or instantaneous. Said two instructions are carried out on the front panel of the device. In case the operation as continuous discharge is selected, the microcomputer operates lighting a luminous indicator of the modality of working, opens the discharge solenoid valve outflowing the air freely and presents the pressure prevailing in the reservoir. In case the operation as instantaneous discharge is selected, it lights the corresponding luminous indicator, keeps closed the solenoid valve, presents the pressure in the reservoir and allows only the outflow of air in an instantaneous way when the discharge push button placed in the applicator is actuated. The information proceeding from the push button is to close the circuit and goes to one of the inputs of instructions of the microcomputer. With the actuation of the push button the reservoir is discharged onto the patient while the push button remains depressed.

3. When the device is connected to the network, the microcomputer initiates the process of starting, opening the solenoid valve and discharging the reservoir in case there is a pressure in the same, till a fixed level, starts the motor that is ready when the same has reached said level and shuts in a continuous way the solenoid valve, being prepared for its use within a time evaluated at most 6 secs.

As an example of a non limiting embodiment, an actual assembling, which will allow a better understanding of the invention, will be described and illustraded in the enclosed drawings, in which:

figure 1 represents diagramatically a possible simplified embodiment,

figure 2 illustrates a pneumatic diagram of a modified device,

figure 3 represents a block diagram of the electronic control circuit of the device according to figure 2,

figures 4, 5 and 6 represent a front elevation, a rear elevation and a bottom plant of an actual embodiment,

figure 7 represents a plant of distribution of the various components inside the casing of the device.

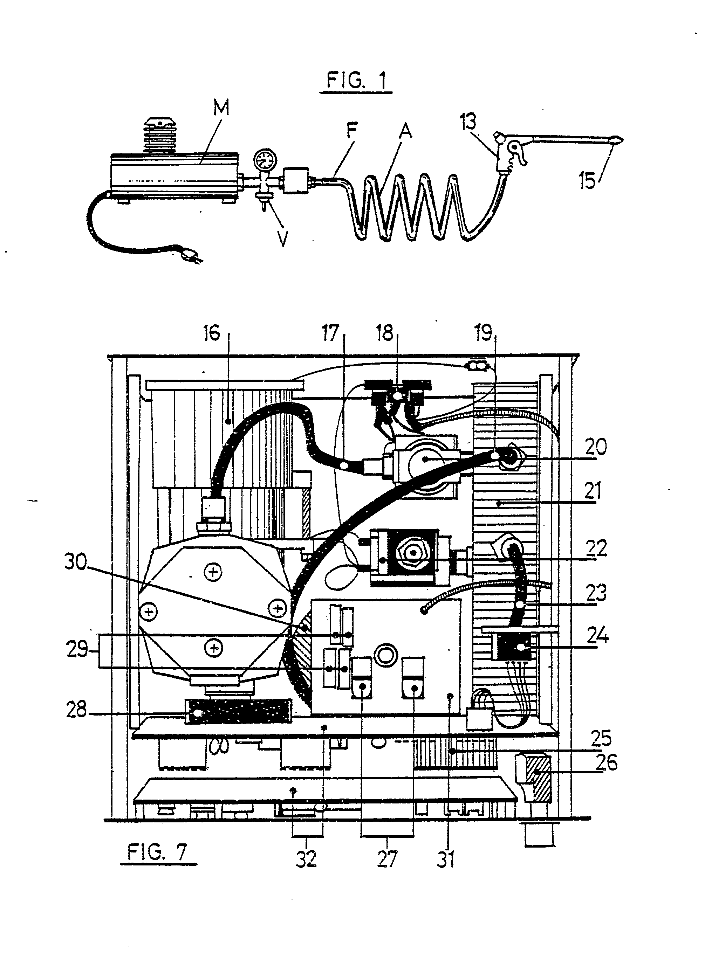

As can be seen in figure 1, the isopressor comprises a compressor M having a low capacity, actuated by means of an electrico motor connectable to the network through a cable and a corresponding plug. At the outlet of the compressor M a pressure gauge, indicating the pressure of the air supplied, is provided, as well a pressure reducer or a pressure reducing valve V. The device comprises further a filter for the separation of the possible particles which can be entrained by the air supplied by the compressor M. At the exit of the filter F a flexible and resistant hose A is connected, at whose free end an exhaust valve 13 is plugged, having the shape of a manual spray run, for example. The outlet of the valve is connected to a flexible hose ending with a nozzle 15 for the administration of the treatment.

The hose will have a sufficient length to allow the required mobility of the nozzle and the gun and to have at the same time the function of a pressurized air accumulator, in order to have at any moment the availability of pressurized air for its supply through the nozzle in a continuous way or as blasts.

In the circuit between the compressor and the gun a volume containing the treatment to be administred can be placed.

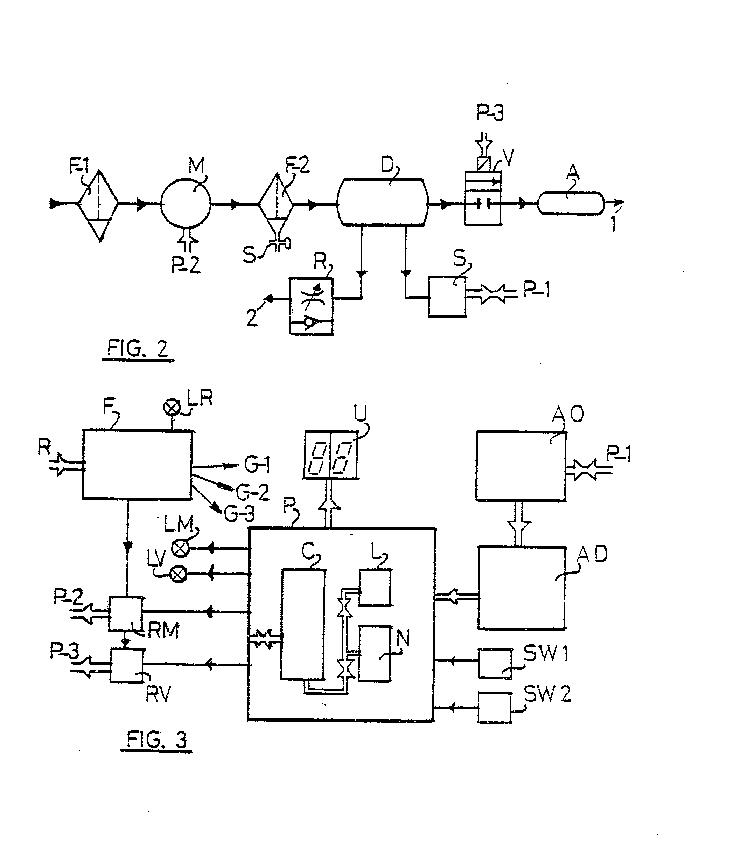

In figure 2 it is shown a pneumatic diagram of the device. The compressor M operates when it is fed with electric current through the connection P-2 from the control equipment with microcomputer. It receives the air through the filter F-1, compresses the same and directs it in the second dry filter F-2, which includes a blow-off valve S for the water, which is accessible from the exterior, in order to separate the water condensed during its operation. From the dry filter it goes to the reservoir D, into which the air is accumulated at the pressure manually selected in the regulator R; the excess air is automatically expelled from the outlet 2 of the regulator in order to mantain inside the reservoir a constant pressure.

To the reservoir a sensor or transductor S is connected, which receives an excitation current from the electronic control circuits, to which it sends as well the transducer signals through the cables P-1. With this sensor the pressure inside the reservoir is continuously measured with a precision higher than with the pressure gauge of the mechanical type.

At the outlet of the air reservoir the exhaust valve V is arranged, which has the function of transferring the compressed air to the air accumulator A arranged in this case in the same applicator, in the other end of which a mouth 1 is arranged, which receives the nozzle for the nose-treatment.

To the exhaust valve arrives the operating voltage through the conductions of electricity P-3, from the control circuits.

This valve will remain permanently open during the operation in continuous discharge, and opens only during the time of actution upon a push button placed on the applicator, when the device must operate with an instantaneous discharge.

In figure 3 a block diagram of the electronic control circuit is illustrated.

With P the microcomputer bloc is illustrated; in this are housed the CPU C, a memory bloc EP-ROM N and a "Lach" for the reading-out of programs and interpretations. To the microcomputer arrive instructions and signals. There arrive instructions relating to the operation mode for the continuous discharge or the instantaneous discharge, both coming from a simple switch identified as SW1. There arrives a discharge instruction according to the operations as a continuous discharge through the closing of a push button identified as SW2 and arranged into the accumulator or in this case the applicator identified as A in figure 1.

The arriving signals come from the pressure sensor or pressure-voltage trasductor, for which a spacing bridge is used, fed with constant current from the circuit identified as AO, and in turn the bridge supplies a voltage, as a function of said current and of the internal pressure of the reservoir, to the same circuit, that supplies it after its convenient amplification and stabilization to an analogic-to-digital converter, which renders it assimilable for the microcomputer. The signal from the sensor and the sensibilization current are fed through the conductors identified as P-1 between AO and S.

The digital signal at the exit of converter AD is identified by the microcomputer which transforms it in readable digits in the numerical indicator U, in this case identified as seven segment digits.

In its peripheral circuitry, the microcomputer is provided with every auxiliary means usual in these devices, such as quart crystal, impedance adapters and discrete components etc.

The auxiliary means of the microcomputer and of the circuits illustrated in blocs consisting in integrated circuits and discrete components of common use will not be represented since there are different single ebomdiments which bring to the same results and are of convential use in electronics.

Subject to the instructions received and the above-mentioned operation modes, the microcomputer acts upon relays or transformation devices of instructions in power signal through which, as represented in figure 2, the compressor and the solenoid valve are actuated.

In the case of the compressor, this will be actuated through the power circuit RM and the electric line P-2.

In the case of the exhaust solenoid valve, this will be actuated through the power circuit RV and the line P-3.

According to how the operation switch SW1 has been actuated, a luminous indicator LM will light to signal the continuous discharge or LV to signal the instantaneous discharge.

All the group of above-mentioned circuits is fed through the electric network R by means of an input source F, in which the different voltages of direct current G-1, G-2 and G-3 are produced, comprising the intrinsic elements of input sources for stabilized direct current.

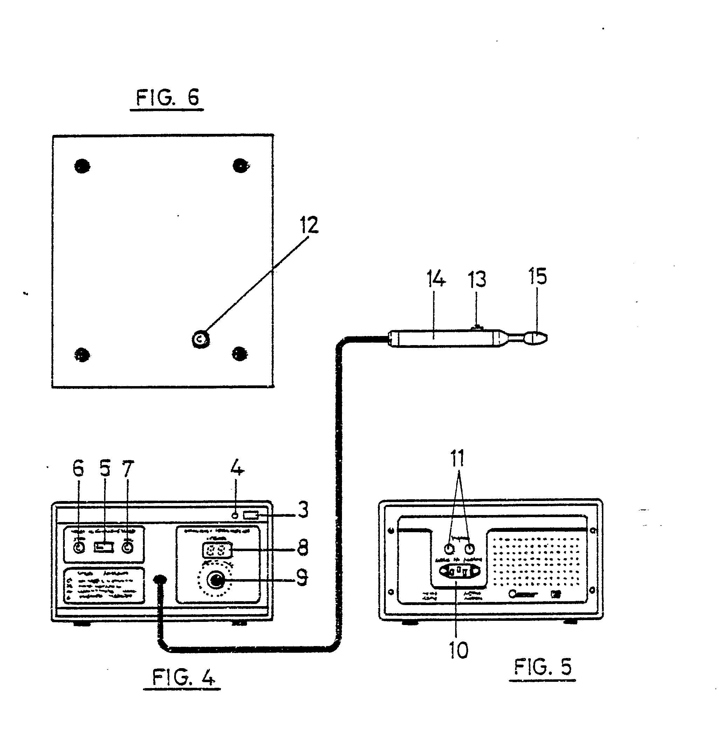

In figures 4, 5 and 6 a front elevation, a rear elevation and a bottom plant are represented, in which the different references are: 3 the start switch; 4, the start luminous indicator; 5, the switch for the selection of the operation mode; 6, the luminous indicator for the instantaneous discharge; 7, the luminous indicator for the continuous discharge; 8, the numerical indicator; 9, the control and adjustmente scale for the internal pressure; 10, the connector to the network; 11, the safety fuses; 12, the port of the blow-off valve; 13, the push button for the release of the instantaneous discharge; 14, the applicator and simultaneusly the accumulator; and 15, the detachable nozzle for the applicator.

Figure 7 represents a drawing in which it can be noted the arrangement in plant inside the isopressor casing of the different elements forming the same; 16, the compressor; 17, the connection of the compressor to the dry filter; 18, the current tap; 19, the connection of air from the reservoir to the applicator; 20, the filter for dry air; 21, reservoir of air; 22, the discharge solenoid valve; 23, the connection of air from the reservoir to the transductor; 24, the transuctor; 25, control and regulation of the pressure; 26, connection switch to the network; 27, 28, 29 and 30 various electronic components on the panel of printed circuit 31 and 32.

Having fully described the nature of the invention, as well as the way to practice the same, it should be noted that the previously set forth arrangements are subject to changes in details not altering the main principle.

## Claims

1. A tympanic isopressor, characterized in that it comprises an air compressor having a low capacity, an air reservoir-accumulator connected to the compressor, an application nozzle connected to the reservoir-accumulator, a pressure regulator, an electronic pressure gauge for the reservoir, a solenoid valve for the control of the output of air from the reservoir to a decompression volume, an air filter for the inlet to the compressor and another for the outlet from said compressor with a blow-off valve, and a microcomputer with auxiliary circuitry, the pressure regulator being connected to a pressure selector arranged in a control panel, and the pressure gauge comprising a pressure transductor, aided by a peripheric electronic circuitry to the microcomputer and comprising a digital indicator arranged in the control panel as well; and the solenoid valve being controlled by the microcomputer, from an instruction given by the control panel for the selection of the continuous or instantaneous discharge, being the outlet for the instantaneous discharge further controlled by a push button preferibly arranged in the applicator, the control panel further comprising indicators of the operation and of the selected discharge system.

2. Isopressor as claimed in claim 1, characterized in that the pressure transductor is mounted in a first auxiliary socket of the reservoir-accumulator and the pressure regulator in a second auxiliary socket of said reservoir.

FIG. 1

FIG. 7

FIG. 2

FIG. 3

FIG. 6

12

14  13  15

6  5  7  4

3

8

9

11

10

FIG. 4

FIG. 5

# EUROPEAN SEARCH REPORT

## DOCUMENTS CONSIDERED TO BE RELEVANT

EP 86106163.8

| Category | Citation of document with indication, where appropriate, of relevant passages | Relevant to claim | CLASSIFICATION OF THE APPLICATION (Int. Cl.4) |
|---|---|---|---|
| A | SOVIET INVENTIONS ILLUSTRATED, section PQ, week C 04, March 5, 1980<br><br>DERWENT PUBLICATIONS LTD., London P 32, page 8<br><br>  * SU-662 091 (GOLDSHTEIN M A) *<br><br>-- | 1 | A 61 F 11/00<br><br>A 61 F 2/18 |
| A | SOVIET INVENTIONS ILLUSTRATED, section PQ, week D 50, January 27, 1982<br><br>DERWENT PUBLICATIONS LTD., London P 32, P 33, page 5<br><br>  * SU-812 287 (LUMUMBA UNIV) *<br><br>-- | 1 | |
| A | WO - A1 - 83/02 556 (DENSERT)<br><br>  * Claims 1-5,7,8; fig. 3 *<br><br>---- | 1 | TECHNICAL FIELDS SEARCHED (Int. Cl.4)<br><br>A 61 F |

The present search report has been drawn up for all claims

| Place of search | Date of completion of the search | Examiner |
|---|---|---|
| VIENNA | 11-08-1986 | MIHATSEK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

  & : member of the same patent family, corresponding document